Europäisches Patentamt

European Patent Office

Office européen des brevets

(11) Veröffentlichungsnummer: 0 032 356
A1

## (12) EUROPÄISCHE PATENTANMELDUNG

(21) Anmeldenummer: 80730084.3

(22) Anmeldetag: 29.12.80

(51) Int. Cl.³: A 61 N 1/04

(30) Priorität: 28.12.79 DE 2952818

(43) Veröffentlichungstag der Anmeldung:
22.07.81 Patentblatt 81/29

(84) Benannte Vertragsstaaten:
AT CH DE FR GB IT LI NL SE

(71) Anmelder: BIOTRONIK Mess- und Therapiegeräte
GmbH & Co Ingenieurbüro Berlin
Sieversufer 8
D-1000 Berlin 47(DE)

(72) Erfinder: Karr, Dieter E.,Dipl.-Phys.
Hans-Reyhing-Strasse 47
D-7250 Leonberg(DE)

(74) Vertreter: Christiansen, Henning, Dipl.-Ing.
Unter den Eichen 108a
D-1000 Berlin 45(DE)

(54) Elektrode für einen implantierbaren Herzschrittmacher.

(57) Bei dieser Elektrode für einen Herzschrittmacher, welcher einen gemeinsamen Anschluß für seinen Stimulationsausgang und seinen Eingangsverstärker aufweist, sind mindestens eine Stimulationsfläche (6) und mindestens eine galvanisch von der Stimulationsfläche getrennte Sensing-Fläche (7) vorgesehen, wobei die Stimulationsfläche elektrisch leitend mit dem herznahen Ende der Elektrodenzuleitung (9) verbunden ist, während die Sensingfläche über einen Bereich (Element 8) mit dem herznahen Ende der Elektrodenzuleitung elektrisch verbunden ist, welcher für die Stimulationsimpulse einen elektrischen Widerstand bildet, der groß ist gegenüber dem Ausgangswiderstand des Herzschrittmachers bei Impulsabgabe und klein in bezug auf dessen Eingangswiderstand.

Fig.1

BIOTRONIK Meß- und Therapiegeräte    29. Dezember 1980
GmbH & Co Ingenieurbüro Berlin
1000 Berlin

B79.40-EU

---

Elektrode für einen implantierbaren Herzschrittmacher

---

B e s c h r e i b u n g

Die Erfindung betrifft eine Elektrode für einen implantierbaren Herzschrittmacher, welcher einen gemeinsamen Anschluß für seinen Stimulationsausgang und seinen Eingangsverstärker aufweist.

Da die Baugröße von implantierbaren Herzschrittmachern angesichts der Mikrominiaturisierung der verwendeten Elektronikschaltungen in zunehmendem Maße von der Größe der verwendeten Energiequellen abhängig ist, gelingt eine weitere Verkleinerung nur dann, wenn die Batterien kleiner bemessen werden können. Da die geforderte Speicherfähigkeit für elektrische Energie jeweils einen nicht wesentlich herabsetzbaren Mindestraum bedingt, ist die Verkleinerung der Baugröße von Herzschrittmachern letztlich davon abhängig, daß es gelingt, den bei der Stimulation aufzuwendenden Strom- bzw. Spannungsbedarf zu verringern. Ein verringerter Energiebedarf ermöglicht andererseits auch – bei gleichbleibender Größe der Energiespeicher – eine entsprechende Verlängerung der Betriebsdauer des Schrittmachers.

Bei unipolaren Elektroden, die bei Schrittmachern der oben angegebenen Art verwendet werden, dient eine gemeinsame Leitung zur Übertragung der Stimulationsimpulse vom Schrittmacher zum Herzen und der den Schrittmacher mit den Herzaktionen synchronisierenden Signale vom Herzen zum Schrittmacher. Die Signalabgabe und -aufnahme am herzseitigen Ende der Zuleitung erfolgt über eine ebenfalls gemeinsame Fläche, wobei für die Dimensionierung der Fläche für die beiden unterschiedlichen Funktionen verschiedene Gesichtspunkte maßgeblich sind. Während für Stimulationszwecke eine kleine Elektrodenoberfläche ausreichend ist, sollte die Fläche zur Signalaufnahme (Sensing-Fläche) relativ groß bemessen sein, um den Elektrolytwiderstand klein zu halten.

Eine Elektrode, die diese Anforderungen erfüllt, ist aus der veröffentlichten US-Patentanmeldung B 535 466 bekannt. Diese Elektrode, bei der die unterschiedlichen Größen der bei Stimulation und Sensing wirksamen Flächen durch unterschiedliche physikalische Stromausbreitungsmechanismen bei galvanisch miteinander verbundenen Flächen hervorgerufen werden, weist den Nachteil auf, daß die Gestaltung dieser Flächen nicht willkürlich vorgenommen werden kann, sondern den physikalischen Gegebenheiten untergeordnet werden muß.

Der Erfindung liegt die Aufgabe zugrunde, eine Elektrode der vorgenannten Art anzugeben, deren Stimulations- und Sensingfläche geometrisch derart frei gestaltet werden können, daß sie den unterschiedlichen Anforderungen bei der Stimulationsimpulsabgabe und der Aufnahme der Herzantwortsignale jeweils einzeln optimal gerecht werden.

Diese Aufgabe wird durch eine Elektrode mit den im Anspruch 1 angegebenen Merkmalen gelöst.

Die Elektrode ist besonders vorteilhaft mit programmierbaren Herzschrittmachern anwendbar, bei denen sich die minimale zur Aufrechterhaltung der Stimulation erforderliche Impulsamplitude von außerhalb des Körpers einstellen läßt, so daß von den energiesparenden Eigenschaften in vollem Umfang Gebrauch gemacht werden kann. Damit ist eine Verlängerung der Betriebszeit bei gleichbleibender Kapazität der Batterien bis zum Dreifachen erzielbar.

Die Erfindung beruht auf der Erkenntnis, daß eine Anpassung an die unterschiedlichen Erfordernisse bei der Signalübertragung von und zum Herzen nur dann optimal gelöst werden kann, wenn die Stimulations- und die Sensing-Fläche voneinander getrennt sind. Da die Stimulationsfläche zur Verkleinerung des Elektrolyt-Widerstandes eine geringere Oberflächengröße aufweisen sollte, ist ein zusätzliches Element vorzusehen, welches mindestens für die Stimulationsimpulse einen großen Widerstand aufweist. Da die Stimulationsimpulse üblicherweise negatives Potential haben, während die Herzantwortsignale positiv sind, kommt als Widerstandselement auch bevorzugt ein Richtleiter - wie beispielsweise eine Diode mit geringer Schwellspannung - in Betracht, der für die Polarität der Stimulationsimpulse in Richtung auf die Sensing-Fläche sperrend ist.

Gemäß einer vorteilhaften Weiterbildung der Erfindung genügt es zum Erreichen des angestrebten Zieles bereits, wenn die Sensing-Fläche über einen ohmschen Widerstand mit dem herznahen Ende der Elektrodenleitung verbunden ist. Weil übliche Herzschrittmacherschaltungen einen Eingangswiderstand in der Größenordnung von 20 Kiloohm aufweisen, ist der Spannungsverlust, der durch einen Widerstand in der Größenordnung von zwei Kiloohm verursacht wird, noch relativ gering, wobei ein solcher Widerstand bereits wirksam das Abfließen einer größeren Ladungsmenge über die Sensing-Elektrode bei der Stimulation verhindert.

Die Gestaltung der zur Signalaufnahme und -abgabe erforderlichen Flächen ist im wesentlichen freigestellt bzw. wird davon bestimmt, wie das Entstehen einer die Reizschwelle des Herzens heraufsetzenden fibrinösen Schicht um die Stimulationsfläche herum verhindert werden kann.

Bei einer bevorzugten Ausführung der Erfindung erfolgt die hochohmige elektrische Verbindung zwischen Sensing-Elektrode und Elektrodenzuleitung über eine einen Stromweg bildende Materialbrücke aus einem Werkstoff, welcher sowohl die gewünschte Körperverträglichkeit als auch die geforderten Leitfähigkeitseigenschaften aufweist. Als günstig kommt dafür ein flexibler leitender Kunststoff, wie beispielsweise Silikonkautschuk, mit einer Beimengung von Graphit oder Silber- und/oder Kupferpulver in Betracht.

Die Verbindung zwischen Sensing-Fläche und Elektrodenzuleitung kann auch über andere Elemente erfolgen, die den Stimulationsimpulsen einen großen Widerstand entgegensetzen, wie sie in den Unteransprüchen angegeben sind.

Ein bevorzugtes Ausführungsbeispiel der Erfindung ist in den Figuren dargestellt und wird nachfolgend näher beschrieben. Es zeigen:

Fig. 1    ein Schaltbild, welches die am Ein- und Ausgang eines Herzschrittmachers vorliegenden elektrischen Verhältnisse und die Verbindung mit der erfindungsgemäßen Elektrode widergibt sowie

Fig. 2    eine Schnittdarstellung des herznahen Endes der erfindungsgemäßen Elektrode.

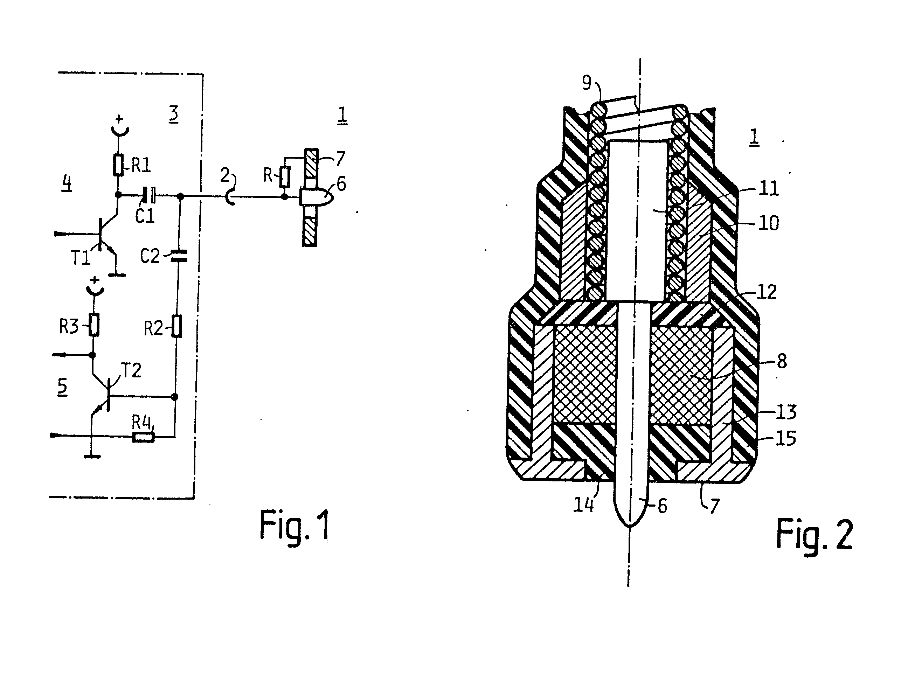

In dem Schaltbild gemäß Fig. 1 ist eine Elektrode 1 mit dem Anschluß 2 eines Schrittmachers 3 verbunden. An den Anschluß 2 gelangen die von einer Endstufe 4 des Herzschrittmachers abgegebenen Stimulationsimpulse. Der Anschluß 2 dient gleichzeitig als Eingang für die vom Herzen abgegebenen Signale, Herzpotentiale, die einer Eingangsstufe 5 zugeführt werden.

Die Ausgangsstufe 4 enthält einen Transistor T1, dessen Eingang mit einem – nicht dargestellten – Impulsgenerator verbunden ist. Weiterhin vorhanden sind ein Kollektorwiderstand R1 von ungefähr 20 bis 30 Kiloohm und ein Koppelkondensator C1 mit einem Wert von ungefähr drei Mikrofarad. Die Eingangsstufe 5 ist über einen Kondensator C2 mit ca. 0,1 Mikrofarad und einen Widerstand R2 von ungefähr 20 Kiloohm mit dem Basisanschluß eines Transistors T2 verbunden, dessen Kollektor den Ausgangsanschluß für weitere Schaltungsteile bildet, welche den nicht dargestellten Impulsgenerator entsprechend dem Herzverhalten des Patienten synchronisieren. Ein Kollektorwiderstand R3 und ein Widerstand R4 zur Einstellung der Basisgleichspannung weisen jeweils Werte von einigen Megohm auf.

Es ist ersichtlich, daß der Eingangswiderstand am Anschluß 2 - von der Elektrode 1 her gesehen - bei gesperrtem Transistor T1 in der Größenordnung von zwanzig Kiloohm liegt.

Die Elektrode 1 besteht in der schematischen Darstellung aus einem kleinflächigen Stimulationsbereich 6, einem größerflächigen Sensing-Bereich 7 und einem Widerstand R.

Geht nun vom Ausgang der Stufe 4 ein Stimulationsimpuls aus, d.h. der Transistor T1 schaltet durch, so ist der Stimulationsbereich 6 der Elektrode 1 über den Kondensator C1 niederohmig mit dem Bezugspotential verbunden – am Aus-

/7

gang 2 entsteht ein negativer Spannungsimpuls. Für die Stimulationswirkung kommt es hierbei im wesentlichen auf die an der Stimulationsfläche 6 entstehenden Feldstärkebedingungen an. Bei einer Herzimpedanz von ungefähr 500 Ohm ist - infolge des Widerstands R von einigen Kiloohm - der über den Sensing-Bereich 7 abschließende Strom gering. Gerade bei größeren Kontaktflächen ist aber bei bekannten Schrittmacherelektroden die Nebenschlußwirkung durch die umgebende Körperflüssigkeit groß. Da zu Stimulationszwecken - nach den für die Elektrotherapie gültigen Gesetzen - lediglich einige wenige Herzzellen stimuliert werden müssen, um eine Herzkontraktion auszulösen, ist dazu ein kleinflächiger Elektrodenbereich ausreichend.

Für die Rückmeldung des Herzverhaltens zum Schrittmacher dient der Sensing-Bereich 7, wobei die R-Zacke in dem mit einem Herzschlag einhergehenden Potentialverlauf (Elektrokardiogramm) über den Eingangsverstärker 5 als Synchronisationssignal ausgewertet wird. Da hier elektrolytische Vorgänge maßgeblich sind, ist mit einer Vergrößerung der Sensing-Fläche 7 eine proportionale Verkleinerung des Elektrolytwiderstandes verbunden. Zusätzlich unterbleibt auch weitgehend eine schädliche Polarisation der Sensing-Fläche. Bei der Signalaufnahme stellt der Stimulationsbereich 6 - wegen des unterschiedlichen zugrundeliegenden Übertragungsmechanismus - keine Beeinträchtigung für die zur Verfügung stehende Signalspannung dar. Der Spannungsabfall, der durch den Widerstand R hervorgerufen wird, läßt sich unter Berücksichtigung des zusammen mit dem Eingangswiderstand am Anschluß 2 gebildeten Spannungsteiler ermitteln und liegt bei der angegebenen Dimensionierung in der Größenordnung von zehn Prozent. Ein derartiger Spannungsverlust kann bei der Dimensionierung durch eine unwesentliche Vergrößerung der Fläche 7 berücksichtigt werden. Der damit verbundene Mehraufwand ist geringfügig im Vergleich zu der über die Verkleinerung der Fläche 6 erzielte Stromersparnis und von Batteriekapazität und Bauform des Schrittmachers.

Das in Fig. 2 dargestellte Ausführungsbeispiel der erfindungsgemäßen Elektrode soll lediglich eine mögliche Bauform mit koaxialem Aufbau von Stimulations- und Sensing-Elektrode (6 bzw. 7) und einem eingefügten Bereich 8 mit einem großen elektrischen Widerstand für die Stimulationsimpulse als Beispiel wiedergeben. Grundsätzlich kann das angegebene Prinzip unabhängig von der Ausführungsform für eine Vielzahl von Elektrodentypen verwendet werden, wobei die räumliche Gestaltung im Einzelfall von der übrigen Konstruktion der Elektrode abhängt. Dabei sind - je nach der vorgesehenen Art der Anbringung im Herzen - gegebenenfalls zusätzliche Bauelemente vorzusehen, welche eine permanente Fixierung ohne wesentliches Ansteigen der Schwellenspannung im Verlaufe der Betriebszeit des Schrittmachers zum Ziele haben. In allen Fällen ist dabei eine relativ großflächige Sensing-Fläche, die elektrolytisch leitend mit dem Herzgewebe in Verbindung steht, zusätzlich zu einem kleinflächigen Stimulationsbereich vorzusehen.

Als elektrische Verbindung der Elektrode dient eine einpolige Zuleitungswendel 9, welche mit einer Hülse 10 auf ein Element 11 aus einem körperverträglichen metallischen Werkstoff aufgepreßt ist, das an seinem herzseitigen Ende die Stimulationsfläche 6 trägt. Im Anschluß an eine Isolationsscheibe 12 ist auf das Element 6 ein Widerstandskörper 8 aufgeschoben, welcher die elektrische Verbindung für die aufgenommenen, von Herzpotentialen hervorgerufenen Signale von dem die Sensing-Fläche 7 tragenden Element 13 zu dem Element 11 herstellt, das ebenfalls aus elektrisch leitendem Material besteht. Der Widerstandskörper 8 kann in bevorzugter Weise aus einem körperverträglichen Isoliermaterial - wie beispielsweise Silikongummi - bestehen, wobei der notwendige Leitwert durch eine entsprechende Beimischung (Graphit, Silber-, Kupferpulver) erzeugt wird. Durch eine geeignete räumliche Bemessung läßt sich der vorgesehene Widerstand zwischen den Elementen 11 und 13 auf den nötigen Wert einstellen. Der verbleibende vordere Abstandsbereich zwischen den Elementen 11 und 13 wird durch einen Körper 14 aus Isoliermaterial ausgefüllt. Die Elektrode und ihre Zuleitung sind von einem Mantel 15 aus Silikongummi umschlossen.

Andere Alternativen für die Ausgestaltung der erfindungsgemäßen Elektrode sind in der Zeichnung nicht dargestellt, lassen sich jedoch durch geringfügige Änderungen herleiten. Werden die Innenfläche des Elements 13 und die Außenfläche so ausgebildet, daß sie einander in dichtem Abstand gegenüberliegende Bereiche mit einem dazwischenliegendem Dielektrikum (das an die Stelle des Bereichs 8 tritt) aufweisen, so ist durch die derart gebildete Kapazität (von vorzugsweise im wesentlichen einem Mikrofarad) die Übertragung der Stimulationsimpulse zur zur Sensing-Fläche 7 dann wirksam herabgesetzt, wenn die Steigung der vom Schrittmacher abgegebenen Stimulationsimpulse gering ist im Vergleich zum Verlauf des Herzpotentials.

Die erfindungsgemäße Elektrode läßt sich in weitem Umfang an die übrigen Konstruktionserfordernisse der Elektrode anpassen: so kann an die Stelle des Bereichs 8 auch ein Elektrolyt treten, welches eine ausreichende Dämpfung der Stimulationsimpulse in Richtung zur Sensing-Elektrode hervorruft, die aufgenommenen Herzpotentiale (bezogen auf den Eingangswiderstand des Schrittmachers) jedoch nur möglichst geringfügig herabsetzt. Bei einer bevorzugten Ausführung der Elektrode wird der Elektrolyt durch im implantierten Zustand der Elektrode eindringende Körperflüssigkeit gebildet, wenn nämlich der Bereich 8 für Körperflüssigkeit durchlässig, so z.B. leer ist und der Körper aus Isoliermaterial 14 geeignete Durchlässe aufweist bzw. ganz entfallen kann, wenn anderweitig für eine geeignete Befestigung der Sensing-Fläche 7 gesorgt ist.

Bei einer anderen bevorzugten Ausführung der Elektrode ist der Bereich 8 durch einen Richtleiter ersetzt, der in Richtung von dem herznahen Ende der Elektrodenzuleitung zur Sensingfläche für die Stimulationsimpulse einen großen elektrischen Widerstand aufweist.

Je nach den Anforderungen an die Stromersparnis und den Widerstandsverhältnissen am Schrittmacheranschluß kommen gegebenenfalls auch Elemente mit größeren Widerstandswerten in Richtung zur Sensing-Fläche 7 in Betracht als sie vorstehend angegeben wurden.

\* \* \* \* \*

Patentansprüche

1.    Elektrode für einen implantierbaren Herzschrittmacher, welcher einen gemeinsamen Anschluß für seinen Stimulationsausgang und seinen Eingangsverstärker aufweist, d a d u r c h   g e k e n n z e i c h n e t ,   daß mindestens eine Stimulationsfläche (6) und mindestens eine galvanisch von der Stimulationsfläche getrennte SensingFläche (7) vorgesehen sind, daß die Stimulationsfläche elektrisch leitend mit dem herznahen Ende der Elektrodenzuleitung (9) verbunden ist, während die Sensingfläche über einen Bereich (Element 8) mit dem herznahen Ende der Elektrodenzuleitung elektrisch verbunden ist, welcher für die Stimulationsimpulse einen elektrischen Widerstand bildet, der groß ist gegenüber dem Ausgangswiderstand des Herzschrittmachers bei Impulsabgabe und klein in bezug auf dessen Eingangswiderstand.

2.    Elektrode nach Anspruch 1,   d a d u r c h   g e k e n n z e i c h n e t ,   daß es sich bei dem Bereich (9) um ein Element (R) mit im wesentlichen ohmschen Widerstand handelt.

3.    Elektrode nach einem der vorangehenden Ansprüche, d a d u r c h   g e k e n n z e i c h n e t ,   daß der ohmsche Widerstand des Elements beim Anschluß an einen Herzschrittmacher mit einem Eingangswiderstand in der Größenordnung von zwanzig Kiloohm im wesentlichen zwei Kiloohm beträgt.

4.    Elektrode nach einem der vorangehenden Ansprüche, d a d u r c h   g e k e n n z e i c h n e t ,   daß es sich bei dem Bereich (8) um ein Element handelt, welches zwei in· einem Abstand voneinander gegenüberliegende, jeweils mit dem herznahen Ende der Elektrodenzuleitung bzw. der Sensingfläche galvanisch verbundene Flächen aufweist, die einen elektrischen Kondensator bilden.

5.    Elektrode nach Anspruch 4, d a d u r c h   g e - k e n n z e i c h n e t ,   daß die Kapazität des Kondensators bei Anschluß an einen Herzschrittmacher mit einem Eingangswiderstand in der Größenordnung von zwanzig Kiloohm im wesentlichen ein Mikrofarad beträgt.

6.    Elektrode nach einem der vorangehenden Ansprüche, d a d u r c h   g e k e n n z e i c h n e t ,   daß es sich bei dem Bereich (8) um ein Element handelt, welches zwischen zwei in einem Abstand voneinander gegenüberliegenden, jeweils mit dem herznahen Ende der Elektrodenzuleitung bzw. der Sensingfläche galvanische verbundenen Flächen eine elektrolytisch leitende Brücke bildet.

7.    Elektrode nach einem der vorangehenden Ansprüche, d a d u r c h   g e k e n n z e i c h n e t ,   daß der Bereich (8) aus einer Materialbrücke aus einem Werkstoff geringer Leitfähigkeit besteht, welche zwischen dem die Stimulationsfläche (6) und dem die Sensing-Fläche (7) tragenden Element (11 bzw. 13) angeordnet ist.

8.    Elektrode nach Anspruch 7, d a d u r c h    g e -
k e n n z e i c h n e t ,    daß die Materialbrücke aus
einem zwischen dem die Stimulationsfläche (6) und dem die
Sensing-Fläche (7) tragenden Elementen (11 bzw. 13) koaxial angeordneten ringförmigen Element (8) besteht.

9.    Elektrode nach einem der Ansprüche 7 oder 8, d a -
d u r c h    g e k e n n z e i c h n e t ,    daß das Element (8) aus Silikonkautschuk mit einer Beimischung zur
Erhöhung der Leitfähigkeit besteht.

10.    Elektrode nach Anspruch 9, d a d u r c h    g e -
k e n n z e i c h n e t ,    daß die Beimischung aus Kupfer, Silber und/oder Graphit besteht.

11.    Elektrode nach einem der Ansprüche 6 bis 10, d a -
d u r c h    g e k e n n z e i c h n e t ,    daß die elektrolytisch leitende Brücke aus einem Bereich gebildet
wird, der im implantierten Zustand der Elektrode für Körperflüssigkeit zugänglich ist.

12.    Elektrode nach Anspruch 1, d a d u r c h    g e -
k e n n z e i c h n e t ,    daß der Bereich durch einen
Richtleiter gebildet wird, der in Richtung von dem herznahen Ende der Elektrodenzuleitung zur Sensingfläche für
die Stimulationsimpulse einen großen elektrischen Widerstand aufweist.

* * * * *

**Fig. 1**

3
4
+ R1
C1
T1
R3
C2
R2
5
T2
R4
2
R
7
6
1

**Fig. 2**

9
1
11
10
12
8
13
15
14
6
7

Europäisches
Patentamt

**EUROPÄISCHER RECHERCHENBERICHT**

0032356

Nummer der Anmeldung

EP 80 73 0084

| Kategorie | EINSCHLÄGIGE DOKUMENTE Kennzeichnung des Dokuments mit Angabe, soweit erforderlich, der maßgeblichen Teile | betrifft Anspruch | KLASSIFIKATION DER ANMELDUNG (Int. Cl.¹) |
|---|---|---|---|
| X | US - A - 3 977 411 (RESEARCH)  * Spalte 2, Zeilen 6-24; Spalte 3, Zeile 64 - Spalte 4, Zeile 33 *  -- | 1-3,12 | A 61 N 1/04 |
|  | FR - A - 2 171 327 (CORDIS)  * Seite 1, Zeile 31 - Seite 2, Zeile 8 *  -- | 1 |  |
|  | DE - A - 1 951 714 (HUGO SACHS)  * Seite 2, Abschnitt 4 *  -- | 6 | RECHERCHIERTE SACHGEBIETE (Int. Cl.³)  A 61 N 1/04 |
|  | US - A - 2 988 663 (GENERAL MOTORS)  * Spalte 1, Zeilen 36-45 *  -- | 7,8 |  |
|  | DE - A - 2 822 829 (LAMPADIUS)  * Anspruch 1; Seite 8, Abschnitt 2 *  -- | 9,10 |  |
|  | DE - A - 2 702 240 (CASE)  * Seite 11, Abschnitt 2; Seite 12, Abschnitte 3,4; Seite 16, Abschnitt 2 *  ---- | 11 | KATEGORIE DER GENANNTEN DOKUMENTE  X: von besonderer Bedeutung  A: technologischer Hintergrund  O: nichtschriftliche Offenbarung  P: Zwischenliteratur  T: der Erfindung zugrunde liegende Theorien oder Grundsätze  E: kollidierende Anmeldung  D: in der Anmeldung angeführtes Dokument  L: aus andern Gründen angeführtes Dokument  &: Mitglied der gleichen Patent-familie, übereinstimmendes Dokument |

Der vorliegende Recherchenbericht wurde für alle Patentansprüche erstellt.

| Recherchenort | Abschlußdatum der Recherche | Prüfer |
|---|---|---|
| Den Haag | 02-04-1981 | SIMON |

EPA form 1503.1 06.78